# EUROPEAN PATENT APPLICATION

(11) **EP 4 104 754 A1**
(43) Date of publication of application: **21.12.2022**
(21) Application number: 21180481.0
(22) Date of filing: 18.06.2021
(51) Int. Cl.: A61B 5/01, A61B 5/107, A61B 5/00, G09B 23/30, A61B 18/00, G01J 5/00

(54) **SYSTEM FOR DETERMINING AN INTERNAL TEMPERATURE**

(71) Applicant: Elmedix NV, 3001 Leuven (BE)
(72) Inventor: Van den Bossche, Johan, B-3210 Linden (BE); Bogers, Johannes, B-2530 Boechout (BE); Brancato, Luigi, B-3010 Leuven (Kessel-Lo) (BE)
(74) Representative: IPLodge bv

(57) **Abstract**

The invention pertains to a system for determining an internal temperature at a predetermined location in a human or animal body, comprising a plurality of temperature sensors for measuring surface temperatures over an area of the body at a surface location on the body; an input for receiving at least one value indicative of: a geometric parameter, an anatomical parameter or a physical property of said human or animal body; an environmental property of the human or animal body; for use in a thermal model of said human or animal body; a processor for calculating the internal temperature at the predetermined location based on the surface temperatures and the at least one input value, by use of said thermal model of a human or animal body; and an output for outputting the calculated internal temperature. The invention also pertains to a method for use in same.

## Description

### Technical field of the invention

The present invention relates to the field of determining an internal temperature at a predetermined location in a human or animal body. The present invention specifically relates to a system for determining an internal temperature at one or more locations in the human or animal body and a corresponding method for use in same, a plurality of temperature sensors for use in such system, and a thermal treatment device comprising such system.

### Background of the invention

In various applications, it is desirable to obtain accurate temperature measurements of specific locations in the animal or human body, such as specific organs, e.g. in the liver, a kidney or the brain.

For example, a body suffering from hypothermia, wherein a body core temperature drops below 36°C, runs an increased risk of surgical wound infection and increased blood loss. It was found that under such conditions, the brain is the most critical temperature sensitive tissue, the temperature of which can differ significantly from the temperature of internal body organs (A Novel Compact Microwave Radiometric Sensor to Noninvasively Track Deep Tissue Thermal Profiles, Maccarini etal., https://jdc.jefferson.edu/radoncfp).

Moreover, a body subjected to thermal treatment may undergo forced elevation of the body temperature to a level that is close to the upper tolerable limit, above which organ damage may occur. By deliberately inducing a hyperthermia condition in a body using drugs and/or medical devices, cancerous cells can be overheated, e.g. at a temperature of 41.5°C. Thus, controlled thermal treatment can be used as a primary or adjuvant treatment of cancer. This approach may be particularly useful for treating cancers that are widely distributed in the body. In such procedure, it is important to ensure that there are no hot spots in the body, which could potentially lead to irreversible damage, and ultimately even death, of the subject. For example, the liver has a high metabolic rate, and may therefore be prone to overheating when the whole body is brought to an elevated temperature. Such overheating could lead to acute liver failure.

Therefore, a need exists in the art for accurate organ temperature monitoring.

It is known in the art to measure temperatures in the body invasively, e.g. by inserting temperature sensors in the body to monitor the temperature at various locations during a thermal treatment procedure. For example, a temperature sensor on the tip of a needle or catheter may be inserted into an organ of interest. However, this type of sensors has the disadvantage of being cumbersome to install. Insertion into the organ of interest also implies a health risk, e.g. due to potential bleeding, tissue damage and/or infection of the organ. This risk may be particularly high for organs that are particularly sensitive or perfused, such as the liver, the kidney or the brain.

For example, brain tissue is typically damaged by a needle over a region around the needle that has the same dimensions as the needle, such that serious and deterministic damage is inflicted on each insertion of a needle. Such risks may be particularly relevant for prolonged use of the invasive sensor, e.g. during a long procedure of 4 to 26 hours, e.g. 8 to 12 hours, as would be typically the case for thermal treatment procedures. Furthermore, the time required for introducing the sensors into the target organs or tissues may prolong the total duration of the procedure for which the temperature measurements are to be used and may particularly increase the time under narcosis for the patient.

Another disadvantage includes the risk of sensor displacement by movement of the needle or catheter out of the target organ. Obviously, when the temperature readings are used for safeguarding against organ failure due to overheating or similar health risks, inaccurate measurements due to a shift in the sensor position could have severe, even lethal, consequences. This also relates to the disadvantage of not being able to visually confirm the correct positioning of the sensor in the organ easily.

Non-invasive alternatives are also known in the art. For example, magnetic resonance imaging may be used to measure the temperature of an organ. Unfortunately, the accuracy is disadvantageously low, e.g. about +/-1°C (In vivo evaluation of multi-echo hybrid PRF/T1 approach for temperature monitoring during breast MRgFUS treatments, Todd et al., doi: 10.1002/mrm.24976), for the purpose of thermal treatment monitoring, e.g. which may require an accuracy of about one order of magnitude higher, e.g. about +/- 0.1°C. Moreover, the temporal resolution of MRI temperature measurements, e.g. about one measurement per hour, may be disadvantageously low, e.g. too low for thermal treatment monitoring. Other disadvantages of this approach include a high cost (both of operation and of investment in equipment), and high requirements for compatibility (e.g. no ferromagnetic materials and/or no electric devices in the MRI cavity).

In the aforementioned publication by Maccarini et al., a non-invasive core temperature thermometer using a small microwave radiometric sensor on the skin surface is used to measure thermal radiation emitted from brain tissue in the 1.1-1.6 GHz band. Small temperature changes with an accuracy of better than 0.5 °C in the deep brain could be tracked, independent of temperatures of the overlying scalp and skull.

The patent application documents WO2017/204733A1 and US2017/100042A1 relate to the estimation of a core body temperature using a heat flux measurement and a temperature measurement on the skin. A reference measurement of the core body temperature is used for calibration.

US 2013/331728A1 discloses a device for the non-invasive measurement of the core temperature of a specific organ, such as the liver or the heart. The device comprises a differential temperature sensor for measuring a temperature difference over a thermal mass, an absolute temperature sensor for measuring an external temperature and a heating element. The core temperature of the organ is determined by measuring both the differential and absolute temperature twice, once before and once after applying a reference amount of heat via the heating element. The internal temperature of the organ is determined by solving a set of equations representative of a thermal equivalent circuit. However, this approach has the disadvantages of requiring an active heat source and being rather complex.

Based on the above, a need still exists to provide means and methods to determine an internal temperature at a predetermined location in a human or animal body, e.g. in specific organs or tissue, at an accuracy of around 0.1°C, wherein such means and methods are essentially aimed to be non-invasive or minimally invasive.

### Summary of the invention

According to an aspect of the present invention, there is provided a system for determining an internal temperature at a predetermined location in a human or animal body, the system comprising:
- A plurality of temperature sensors for measuring surface temperatures over an area of the body at a surface location on the body;
- an input for receiving at least one value indicative of:
   ∘ a geometric parameter, an anatomical parameter or a physical property of said human or animal body or of a part of said human or animal body;
   ∘ an environmental property of the human or animal body;
   for use in a thermal model of said human or animal body;
- a processor for calculating the internal temperature at the predetermined location based on the surface temperatures and the at least one input value, by use of said thermal model of a human or animal body; and
- an output for outputting the calculated internal temperature.

It is an advantage of the present invention that the (or any) internal temperature of a (or any) biological tissue, at several (or any) locations can be accurately determined. For example, the temperature at the boundary of an organ, or at different depths within the organ can be accurately determined. It has been found that an accuracy of 0.2°C can be guaranteed, generally an accuracy of 0.1 °C can be guaranteed, and under some circumstances an accuracy of 0.05°C is attainable. It is a further advantage that the system and method as described herein are provided to obtain such internal temperature in a non-invasive or minimally invasive manner. Hence, a temperature value can be obtained without having to insert a needle or catheter into an organ which may cause infection, damage and/or internal bleeding in an individual whose health conditions may already be weakened.

In an embodiment of the system according to the invention, said plurality of temperature sensors comprises a plurality of subcutaneous temperature sensors.

It is an advantage of this embodiment that, as a consequence of the choice for subcutaneous temperature sensors, the thermal isolating influence of the skin, can be circumvented. Indeed, said subcutaneous temperature sensors as used herein are typically provided to pierce through the epidermis and dermis layers of the skin and to measure the temperature preferably at the level of the subcutaneous tissue of hypodermis. Typically, the subcutaneous temperature sensors are provided to measure at a depth of 4-7 mm under the skin surface. Hence, the thermally isolating function of the skin can be bypassed and hence thermal influences of the immediate vicinity of the human or animal body. It is a further advantage that any temperature gradients introduced by eventual transpiration or evaporation driven by the sweat produced by sweat glands can also be partially circumvented.

In an embodiment of the system according to the invention, values for said geometric parameters or said anatomical parameters are provided by measuring the body of an individual human or animal by use of Magnetic Resonance Imaging (MRI) or Computer Tomography (CT).

It is an advantage of this embodiment that medical imaging techniques can be used for increasing the accuracy of the system, e.g. by providing real data on corporal dimensions of the body of a specific individual human or animal. Often, such data will already be available, for example as a result of medical examinations for the purpose of treating diseases, e.g. for mapping the size of a tumour.

In an embodiment of the system according to the invention, said thermal model is provided to use a geometric model of the human or animal body, wherein such geometric model is selected from the following:
∘ a one-dimensional model;
∘ a compartmental model of a body anatomy by use of three-dimensional objects, such as cylinders, spheres, cuboids, cubes and the like;
∘ a whole-body generic model for a standard male or female human or animal;
∘ a whole-body specific model for a body of an individual human or animal, using geometric data measured for said body of said individual human or animal; or combinations of said models.

A thermal model is intrinsically related to a geometric representation or model of the human or animal body it wishes to model. It is an advantage of this embodiment that said internal temperature can be determined by solving a thermal model linked to a geometric representation, the complexity and concrete nature of which may be adapted to the location of which a temperature has to be determined. It will be understood by the skilled person that thermal or geometric models may be more appropriate for determining a temperature in some locations, e.g. an abdomen, than others, e.g. in a limb or the brain. The skilled person will be able to select an appropriate model for a given situation, wherein aforementioned thermal or geometric models may furthermore be combined, e.g. a whole body-generic model for modeling thermoregulation in an abdomen combined with a one-dimensional or linear model extending from the skin to a point inside the abdomen.

In an embodiment of the system according to the invention, said geometric data is provided by measuring the body of an individual human or animal by use of Magnetic Resonance Imaging (MRI) or Computer Tomography (CT).

It is an advantage of this embodiment that medical imaging techniques can be used for increasing the accuracy of the system, e.g. by providing real data on corporal dimensions, such as the exact shape of organs or tissue, e.g. the liver, kidney, a lung, the brain, the colon, of the human or animal body.

In an embodiment of the system according to the invention, said system further comprises a second temperature sensor, insertable at or near said predetermined location in the human or animal body, for invasively measuring said internal temperature at said predetermined location in the human or animal body and for further increasing the accuracy of the system.

It is an advantage of this embodiment that a second temperature sensor may be provided, which allows to verify the accuracy of the temperature determination of a predetermined location as described herein. The processor will then as a consequence be adapted to use the temperature measurement of said invasive second temperature sensor to verify and evaluate the accuracy of the internal temperature determined by the system. The processor may be adapted to use said value for refining said determination of the internal temperature and for increasing the accuracy of the system, e.g. by correlating and/or calibrating the determined value for the internal temperature with the value measured by the second temperature sensor. In embodiments, said second temperature sensor is adapted to be inserted in an organ or tissue of interest, e.g. at a first use of the system with an individual body, wherein said processor is further adapted to correlate the internal temperature at the predetermined location determined on the basis of the surface temperatures and the at least one input value with the measured internal temperature provided by said second temperature sensor and/or to calibrate said determined value for the internal temperature by use of the measured internal temperature provided by said second temperature sensor.

In an embodiment of the system according to the invention, said predetermined location is one of the following: liver, colon, kidney, brain.

It is an advantage of this embodiment that the temperature of body parts or zones, which are sensitive to thermal conditions deviating from the norm, such as the liver, colon, kidney and/or the brain may be determined in a reliable way, with an accuracy as mentioned here above.

According to an aspect of the present invention, there is provided a thermal treatment device for temporarily increasing the temperature of a human or animal body comprising said system for determining an internal temperature in accordance with any of the previous claims, and a controller for controlling at least one heater and/or cooler of the thermal treatment device, said controller being adapted for receiving the internal temperature from the output of the system and for taking said internal temperature into account in controlling the at least one heater and/or cooler.

It is an advantage of this embodiment that the accurate and continuous determination of the internal temperature at a point of interest in a body, allows for the use of a thermal treatment on said body. Advantageously, a controller may use the determined internal temperature as an input for controlling the temperature regulation of the body, decreasing the risk for any local overheating in the body.

According to an aspect of the present invention, there is provided a plurality of temperature sensors for use in the system for determining an internal temperature at a predetermined location in a human or animal body in accordance with any of the previous claims.

In an embodiment according to the invention, said plurality of temperature sensors comprises a plurality of subcutaneous temperature sensors.

According to an aspect of the present invention, there is provided a method for determining an internal temperature at a predetermined location in a human or animal body, the method comprising the following steps:
- receiving 101 at least one value indicative of a
   ∘ a geometric parameter, an anatomical parameter or a physical property of said human or animal body or of said predetermined location in the human or animal body;
   ∘ an environmental property of the human or animal body;
   for use in a thermal model of said human or animal body;
- measuring 102 surface temperatures over an area of the body at a surface location on the body;
- calculating 104 the internal temperature at the predetermined location based on the surface temperature and the input value, by use of said thermal model of a human or animal body; and
- outputting 105 the calculated internal temperature.

In an embodiment of the method according to the invention, the method further comprises a step 103 for increasing the accuracy of determining said internal temperature, wherein said step 103 comprises, prior to step 104:
- inserting a second temperature sensor for invasively measuring said internal temperature at said predetermined location in the human or animal body.

In an embodiment of the method according to the invention, the method further comprises the step of
- comparing 106 the calculated internal temperature with the invasively measured internal temperature and refining said calculating of the internal temperature by correlating and/or calibrating the calculated internal temperature with the invasively measured internal temperature.

According to an aspect of the present invention, there is provided a computer program product for carrying out computer program product comprising code means configured to cause a processor to perform the functions of the system as described herein.

These and other aspects of the present invention will be apparent from and elucidated with reference to the embodiment(s) described hereinafter.

The independent and dependent claims describe specific and preferred features of the invention. Features of the dependent claims can be combined with features of the independent claims and with features of other dependent claims as deemed appropriate, and not necessarily only as explicitly stated in the claims.

### Brief Description of the Drawings

FIG 1 illustrates a device in accordance with embodiments of the present invention.
FIG 2 illustrates a method in accordance with embodiments of the present invention.

The drawings are schematic and non-limiting. Elements in the drawings are not necessarily represented on scale, e.g. an element may be exaggerated for illustrative purposes or reduced in scale to keep the drawing clear and comprehensible. The present invention is not necessarily limited to the specific embodiments of the present invention as shown in the drawings. Reference signs in the claims shall not be construed as limiting the scope.

### Detailed description of embodiments

The present invention relates to a system for determining an internal temperature at a predetermined location of interest in a human or animal body. A specific application of the system described herein relates to determining an internal temperature in a tissue of interest or an organ of interest, such as the liver, the stomach, a kidney, a lung, the brain, the colon or any other organ or tissue. The internal temperature may be measured in the organ or at an outer boundary of said organ of interest. As described here above, an accurate estimation of the temperature inside a body is often needed, such as for bodies undergoing thermal treatment, wherein the body is heated to a certain temperature for a period of time, or wherein the body is in a condition of hypothermia, having an overall core temperature of less than 36°C.

The system may be provided to determine such temperature in a non-invasive, in a minimally invasive manner or in an invasive manner.
For the purpose of the invention, the term "non-invasive" relates herein to a medical procedure wherein no break in the skin is created and there is no contact with the mucosa or any internal body cavity beyond a natural or artificial body orifice.
For the purpose of the invention, the term "minimally invasive" relates herein to medical procedures which differ from non-invasive medical procedures in that small incisions in the skin, causing skin break, may occur for inserting sensors for subcutaneous measurements, e.g. subcutaneous temperature measurements. Such small incisions are superficial and typically a few mm deep. There is no contact with the mucosa or any internal body cavity beyond a natural or artificial body orifice.

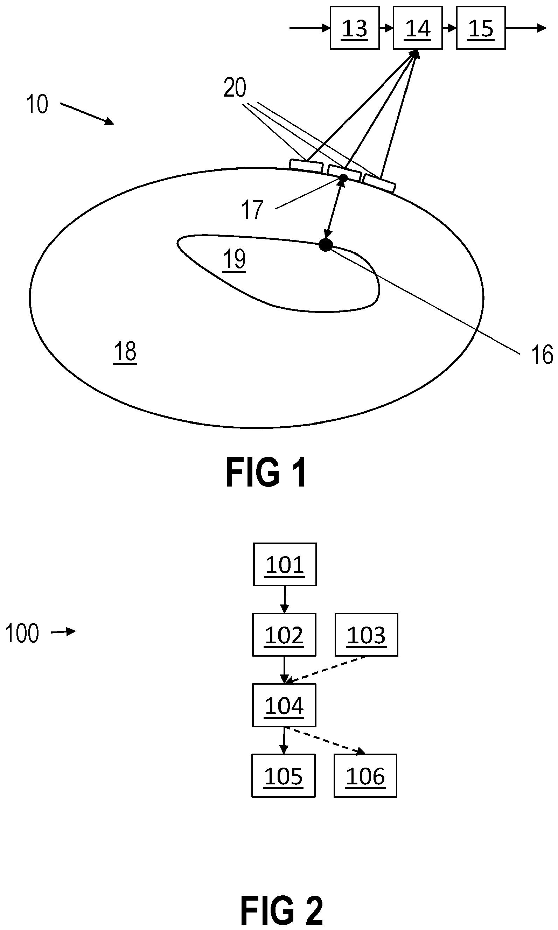

Referring to FIG 1, an exemplary system 10 in accordance with embodiments of the present invention is shown. System 10 is adapted for determining the internal temperature Tint at a predetermined location 16 in the human or animal body 18, such as in an organ 19 of interest, for example an internal temperature at the outer boundary of the organ.

The system 10 comprises a plurality of temperature sensors 20 for measuring surface temperatures over an area of the body at a surface location 17 on the body 18, e.g. on the skin of the body 18. Typically, said area is surrounding said surface location 17.

In embodiments according to the invention, said plurality 20 comprises at least 2, 3, 4, 5 or 6 temperature sensors. Typically, said plurality does not comprise more than 10 temperature sensors. Typically, the temperature sensors are contacted with an area of the body at a surface location 17 on the body 18, in a rectangular or circular shape. The area typically does not exceed 600 cm2. It will be understood that the plurality of temperature sensors 20 covers an area and are located around the surface location 17, which may form a central point for said plurality 20 on the body 18, e.g. on the skin of the body 18.

The surface location 17 is generally located directly above or in the immediate surroundings of the predetermined location 16, e.g. may approximate a closest distance from the predetermined location 16 to the surface of the body. For example, the surface location 17 may be separated from the predetermined location 16 by a layer of skin, e.g. by layers of skin and fat, e.g. by layers of skin, fat and/or muscle. As such, taking into account geometric and anatomical parameters and physical properties of the human or animal body in combination with a body thermal model as well as the temperatures measured by said plurality of temperature sensors 20, an accurate estimation of the internal temperature of a predetermined location of interest is obtainable.

In embodiments according to the invention, the temperature sensors of said plurality 20 may comprise a thermistor, a thermocouple, a thermopile or a silicon bandgap temperature sensor.

In preferred embodiments, said plurality of the temperature sensors 20 comprises a plurality of subcutaneous temperature sensors. Preferably, said plurality of temperature sensors consists of a plurality of subcutaneous temperature sensors. The subcutaneous temperature sensors are provided to pierce through the epidermis and dermis layers of the skin and to measure the temperature preferably at the level of the subcutaneous tissue of hypodermis. Typically, the subcutaneous temperature sensors are provided to measure at a depth of 4-7 mm under the skin surface.

It is to be understood that each of said plurality of temperature sensors 20 has been calibrated in order to provide for a reliable temperature measurement. Typically, each of said sensors has an accuracy of at least 0.1°C, preferably of at least 0.05°C.

The system 10 further comprises an input 13 adapted for receiving at least one value indicative of:
∘ a geometric parameter, an anatomical parameter or a physical property of said human or animal body or of a part of said human or animal body;
∘ an environmental property of the human or animal body;
for use in a thermal model of said human or animal body.

A geometric parameter of a human or animal body or of a part of said human or animal body, such as a part being or including said predetermined location, may refer to the shape and/or dimension of a three-dimensional object for representing a body part. Said three-dimensional object for representing a body part may include a sphere, a cuboid, a cylinder, a cube or any other suitable objects.

An anatomical parameter of a human or animal body or of a part of said human or animal body, such as a part being or including said predetermined location, may refer to a dimension, such as a length, and/or a diameter, and/or a volume and/or a surface area, of a part of the human or animal body, such as an abdomen, a limb, a thorax, a head. The anatomical parameter may for example refer to a dimension, such as a length, and/or a thickness, and/or a diameter, and/or a volume and/or a surface area, of a skin layer, and/or a fat layer, and/or a bone layer, and/or a muscle layer.

A physical property of a human or animal body or of a part of said human or animal body, such as a part being or including said predetermined location, may refer to thermal and/or mechanical properties of an individual material forming a human or animal body, such as skin, and/or muscle, and/or fat, and/or bone, and/or of an individual body structure and/or an organ. The thermal and/or mechanical properties include at least one of density, specific heat, thermal conductivity, basal metabolic rate, basal blood flow, or metabolic heat.

Said geometric parameter, an anatomical parameter or a physical property are provided for use in a thermal model of a human or animal body. It will be clear to the skilled person that the parameter or property that needs to be provided to the input is related to and defined by the nature of thermal model that is used.

In embodiments according to the invention, said at least one value, e.g. a value indicative of an anatomical parameter, may be provided by measuring the body of an individual human or animal by use of Magnetic Resonance Imaging (MRI) or Computer Tomography (CT), by use of ultrasound imaging, by use of a skinfold measurement (e.g. using calipers). Alternatively, said at least one value, e.g. a value indicative of an anatomical parameter, may be provided by use of an imaging system, e.g. a three-dimensional imaging system, using a range detector for determining a distance from at least a point of reference to a plurality of points on the surface of the body, e.g. at a plurality of points on the body. Alternatively, said imaging system may use at least two cameras configured such as to determine an image depth in images obtained by the cameras. For example, the at least two cameras may form a stereographic imaging system. Preferably, the at least two cameras may comprise at least pairs of cameras at a plurality of locations around the body and aimed at the body, such as to enable the processor to construct a surface model of the body.

An environmental property of the human or animal body may refer to the temperature of the immediate vicinity of the body, such as the temperature of the room the body is located in. An environmental property of the human or animal body may further refer to at least one of air humidity in the immediate vicinity of the body, ambient pressure, air velocity, meaning the speed at which air surrounding the body touches or "hits" on the body, or any other property of the direct environment of the body.

Said environmental property is provided for use in a thermal model of a human or animal body. It will be clear to the skilled person that the parameter that needs to be provided to the input is related to and defined by the nature of thermal model that is used.

The system further comprises a processor 14 for calculating the internal temperature at the predetermined location 16 based on the surface temperatures and the at least one input value provided, by use of a thermal model of a human or animal body.

Without limitation, the term "processor", as used herein, may refer to a programmable hardware component, such as a commercially available microprocessor, which, when provided with appropriate instructions (software), performs the calculations described herein. The term "processor" may also refer to a configurable component, such as an FPGA, appropriately configured to carry out said calculations, or a dedicated hardware component, such as an ASIC, designed to carry out said calculations. The "processor" in embodiments of the present invention may also be implemented as a combination of several such components.

It is known that heat transfer in biological tissues is a complex process involving conduction, convection and radiation mechanisms, beside heat generation by metabolic processes. A body thermal model (BTM) can be used for predicting thermal behaviour of the human or animal body. BTMs represent the human body from a thermokinetic point of view and they have been used for modelling the thermoregulation system. The first model that successfully described the effect of metabolic heat generation rate and blood perfusion on temperature distribution in a living tissue was proposed by Henry Pennes in 1948. Ever since, a number of bioheat transfer equations for living tissues have been proposed in literature. Typically, the model will take into account, to different degrees of precision, the heat exchanged between organs by means of conduction, convection and radiation and the effect of blood on thermal distributions in the body.

It has now been found that the combination of surface temperatures measured at an area near a predetermined location 16 of interest with a thermal model, provided with values for parameters specific for said model, provides for an excellent approximation of the temperature at said location of interest.

According to embodiments of the invention, the thermal model is provided to use a geometric model of the human or animal body, wherein such model is selected from the following:
∘ a one-dimensional model;
∘ a compartmental model of a body anatomy by use of three-dimensional objects, such as cylinders, spheres, cuboids, cubes and the like;
∘ a whole-body generic model for a standard male or female human or animal;
∘ a whole-body specific model for a body of an individual human or animal, using geometric data measured for said body of said individual human or animal.

According to embodiments of the invention, said thermal model is provided to use a one-dimensional or linear model as a geometric model.
For the purpose of the invention, the term "one-dimensional model" refers herein to a model wherein heat transfer equations are solved in a single dimension, e.g. by taking into account heat exchanges along a direction between the surface location 17 on the body and the predetermined location 16 of interest in the human or animal body. Typically, the body is reduced to a number of discrete adjacent layers, including a skin layer, muscle layer, fat layer and a bone layer, each with a thickness and specific values for said physical properties. A value for the individual thicknesses of the layers can be derived by one of the techniques, such as MRI or CT, mentioned here above.

According to embodiments of the invention, said thermal model is provided to use a compartmental model of a body anatomy by use of three-dimensional objects, such as at least one of a cylinder, a sphere, a cuboid, a cube and the like, as a geometric model.

Such models are known from literature. "A transient three-dimensional heat transfer model of the human body" (M. Silva Ferreira et al., DOI: 10.1016/j.icheatmasstransfer.2009.03.010) describes the use of elliptical cylinders to approximate body geometry, which is composed of 15 cylindrical elements and includes heat transfer between large arteries and veins.
"Comprehensive evaluation on the heating capacities of four typical whole body hyperthermia strategies via compartment model" (S-H Xiang, doi:10.1016/j.ijheatmasstransfer.2008.04.024) describes a compartmentalization of the human body in 12 body segments (head, neck, thorax, abdomen, two mars, two hands, two legs, and two feet) and a blood compartment. All segments are cylindrical in shape, except for the head which is assumed to be a sphere. Each body segment is further subdivided into four concentric layers (core, muscle, fat and skin).

EP3751577A1, filed by the present applicant, describes a method for predicting and/or simulating temperatures in the body when the body is exposed to a controllable environment. Paragraphs [0065]-[0138] which are incorporated herein by reference, as well as the claims and the figures of said patent application, describe a plurality of equations representative of an equivalent thermal circuit. In embodiments according to the invention, said compartmental model refers to the model described in said paragraphs.

According to embodiments of the invention, said thermal model is provided to use a whole-body generic model for a standard male or female human or animal as a geometrical model. Such models have been developed and are available in online libraries. The ITIS virtual population (https://itis.swiss/virtual-population/) offers whole-body, and region-specific human models as well as animal models. "Population of anatomically variable 4D XCAT adult phantoms for imaging research and optimization" (W.P. Segars et al, doi: 10.1118/1.4794178) discloses whole-body models for the standard male and female adult, including the cardiac and respiratory motions.

According to embodiments of the invention, said thermal model is provided to use a whole-body specific model for a body of an individual human or animal, using geometric data measured for said body of said individual human or animal as a geometric model. In embodiments according to the invention, said geometric data is provided by measuring the body of an individual human or animal by use of Magnetic Resonance Imaging (MRI) or Computer Tomography (CT). Alternatively, said geometric data may be provided by use of an imaging system, e.g. a three-dimensional imaging system, using a range detector for determining a distance from at least a point of reference to a plurality of points on the surface of the body, e.g. at a plurality of points on the body. Alternatively, said imaging system may use at least two cameras configured such as to determine an image depth in images obtained by the cameras. For example, the at least two cameras may form a stereographic imaging system. Preferably, the at least two cameras may comprise at least pairs of cameras at a plurality of locations around the body and aimed at the body, such as to enable the processor to construct a surface model of the body.

It will be appreciated by the skilled person that the geometric data provided by any of the techniques and methods described hereabove for use in a thermal model, are related to, may be similar to or overlap with, or may be the same as the anatomical parameters provided by any of said techniques and methods.

The processor 14 may be adapted for calculating the internal temperature based on advanced numerical simulations using the provided at least one input value, said measured surface temperatures and a thermal model as described herein.

Alternatively, existing software packages may be used for this purpose. Such software packages include, but are not limited to: MATLAB, COMSOL Multiphysics and SIM4LIFE.

In embodiments according to the invention, said system further comprises a second temperature sensor (not shown in FIG.1) provided to be inserted at or near said predetermined location 16 in the human or animal body 18, for invasively measuring said internal temperature at said predetermined location 16 in the human or animal body 18. Said second temperature sensor may be integratable in, or insertable through, a needle or a catheter. Said second temperature sensor provided to be inserted at or near said predetermined location 16 in the human or animal body 18, is preferably adapted for use in combination with said plurality of temperature sensors 20, e.g. for the purpose of increasing the accuracy of determining said internal temperature, for calibrating said plurality of temperature sensors 20 or for further refining said calculating of the internal temperature. According to these embodiments, the processor 14 is then further adapted for increasing said accuracy and/or for further refining said calculating of the internal temperature and/or for calibrating said plurality of temperature sensors 20 using a temperature measurement from said second temperature sensor provided to be inserted at or near said predetermined location in the human or animal body. In embodiments, said second temperature sensor is adapted to be inserted in an organ or tissue of interest at a first use with an individual body, wherein said processor 14 is further adapted to correlate the internal temperature at the predetermined location determined on the basis of the surface temperatures and the at least one input value, with the measured internal temperature provided by said second temperature sensor and/or to calibrate said determined value for the internal temperature by use of the measured internal temperature provided by said second temperature sensor. Hence, the processor 14 may perform such a step during an initial measurement operation.

WO2021111014A1, filed by the present applicant, describes a compact and safe device for accurately measuring the temperature at one or more locations along an insertion path in the human or animal body. Page 7, line 34 - page 16, line 8 which is incorporated herein by reference, relates to a device for measuring a temperature at one or more locations in an organ or tissue inside the human or animal body. In embodiments according to the invention, said second temperature sensor comprises, consists of, or relates to the device for measuring a temperature described on said pages.

The system further comprises an output 15 for outputting the calculated internal temperature. For example, such output 15 may comprise a wireless communication module or a bus interface for outputting a digital signal representative of the internal temperature via a signal wire, and/or a display device for displaying the internal temperature in a human-readable format.

In an embodiment in accordance with embodiments of the present invention, the input 13, the processor 14 and the output 15 may be implemented in software executing on a general-purpose computing device, such as a computer, a tablet or a mobile phone.

In an embodiment in accordance with embodiments of the present invention, an output 15 may be the controller of e.g. a thermal treatment device for the purpose of controlling the body temperature of an individual subjected to thermal treatment, wherein it must be guaranteed that certain parts or zones of the body do not exceed a certain threshold temperature.

In another aspect according to the present invention, there is provided a thermal treatment device for temporarily increasing the temperature of a human or animal body comprising the system for determining an internal temperature as described herein. Such a thermal treatment device may refer to a device for temporarily increasing the overall body temperature of an individual's body, e.g. for a period of at least 4 hours, 8 hours or at least 12 hours, e.g. in the context of a medical treatment, such as the treatment of a tumour. Such devices have been described in WO2019211411 A1. Said thermal treatment device may also refer to a device for treating a body suffering from a condition of hypothermia.

Said thermal treatment device may further comprise a controller for controlling at least one heater and/or cooler, wherein said controller is further adapted for receiving the internal temperature. Hence, the internal temperature determined by the system as described herein functions as input parameter for the controller allowing to accurately steer the body temperature of the individual's bod y. Advantageously, by using the system as described herein, the thermal treatment device may prevent the exceeding of temperature limits in the individual's body.

In another aspect according to the present invention, there is provided a plurality of temperature sensors for use in the system for determining an internal temperature at a predetermined location in a human or animal body in accordance with any of the previous claims.

In another aspect according to the present invention, and referring to FIG. 2, there is provided a method (100) for determining an internal temperature at a predetermined location (16) in a human or animal body (18), the method comprising the following steps:
- receiving 101 at least one value indicative of a
   ∘ a geometric parameter, an anatomical parameter or a physical property of said human or animal body or of said predetermined location in the human or animal body;
   ∘ an environmental property of the human or animal body;
   for use in a thermal model of said human or animal body;
- measuring 102 surface temperatures over an area of the body at a surface location 17 on the body;
- calculating 104 the internal temperature at the predetermined location 16 based on the surface temperature and the input value, by use of said thermal model of a human or animal body; and
- outputting 105 the calculated internal temperature.

Typically, the steps 101, 102, 104, and 105 are performed by a processor 14.

In embodiments according to the invention, the method may also increase the accuracy of determining said internal temperature and comprises a further step 103, which is carried out prior to step 104, and which comprises:
- inserting a second temperature sensor for invasively measuring said internal temperature at said predetermined location in the human or animal body.

In embodiments according to the invention, the method further comprises the step 106 of
- comparing the calculated internal temperature with the invasively measured internal temperature and refining said calculating of the internal temperature by correlating and/or calibrating the calculated internal temperature with the invasively measured internal temperature.

The processor 15 is then further adapted to evaluate and compare the measurement provided by said second temperature sensor as described here above and to correlate both values and/or to perform a calibration. Correlation and/or calibration may be done by e.g. adjusting the calculated internal temperature to the invasively measured internal temperature.

In another aspect according to the present invention, there is provided a computer program product, optionally stored on a computer-readable medium comprising code means configured to cause a processor to perform the functions of the system, the thermal treatment device and the method as described herein.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

## Claims

1. A system (10) for determining an internal temperature at a predetermined location in a human or animal body, the system comprising:
- A plurality of temperature sensors (20) for measuring surface temperatures over an area of the body at a surface location (17) on the body (18);
- an input (13) for receiving at least one value indicative of:
∘ a geometric parameter, an anatomical parameter or a physical property of said human or animal body or of a part of said human or animal body;
∘ an environmental property of the human or animal body;
for use in a thermal model of said human or animal body;
- a processor (14) for calculating the internal temperature at the predetermined location based on the surface temperatures and the at least one input value, by use of said thermal model of a human or animal body; and
- an output (15) for outputting the calculated internal temperature.

2. The system (10) according to claim 1, wherein said plurality of temperature sensors (20) comprises a plurality of subcutaneous temperature sensors.

3. The system (10) according to claim 1 or claim 2, wherein values for said geometric parameters or said anatomical parameters are provided by measuring the body of an individual human or animal by use of Magnetic Resonance Imaging or Computer Tomography.

4. The system (10) according to any one of previous claims, wherein said thermal model is provided to use a geometric model of the human or animal body, wherein such geometric model is selected from the following:
∘ a one-dimensional model;
∘ a compartmental model of a body anatomy by use of three-dimensional objects, such as cylinders, spheres, cuboids, cubes and the like;
∘ a whole-body generic model for a standard male or female human or animal;
∘ a whole-body specific model for a body of an individual human or animal, using geometric data measured for said body of said individual human or animal;
or combinations of said models.

5. The system (10) according to claim 4, wherein said geometric data is provided by measuring the body of an individual human or animal by use of Magnetic Resonance Imaging or Computer Tomography.

6. The system (10) according to any one of previous claims, wherein said system further comprises a second temperature sensor, provided to be inserted at or near said predetermined location in the human or animal body, for invasively measuring said internal temperature at said predetermined location in the human or animal body and for further increasing the accuracy of the system.

7. The system (10) according to any one of previous claims, wherein said predetermined location is one of the following: liver, colon, kidney, brain.

8. A thermal treatment device for temporarily increasing the temperature of a human or animal body comprising said system (10) for determining an internal temperature in accordance with any of the previous claims, and a controller for controlling at least one heater and/or cooler of the thermal treatment device, said controller being adapted for receiving the internal temperature from the output (15) of the system (10) and for taking said internal temperature into account in controlling the at least one heater and/or cooler.

9. A plurality of temperature sensors (20) for use in the system (10) for determining an internal temperature at a predetermined location in a human or animal body in accordance with any of claims 1-7.

10. The plurality of temperature sensors (20) according to claim 9, wherein said plurality of temperature sensors comprises a plurality of subcutaneous temperature sensors.

11. A method (100) for determining an internal temperature at a predetermined location (16) in a human or animal body (18), the method comprising the following steps:
- receiving (101) at least one value indicative of a
∘ a geometric parameter, an anatomical parameter or a physical property of said human or animal body (18) or of said predetermined location (16) in the human or animal body (18);
∘ an environmental property of the human or animal body;
for use in a thermal model of said human or animal body;
- measuring (102) surface temperatures over an area of the body at a surface location (17) on the body;
- calculating (104) the internal temperature at the predetermined location (16) based on the surface temperatures and the input value, by use of said thermal model of a human or animal body; and
- outputting (105) the calculated internal temperature.

12. The method according to claim 11, the method further comprising a step (103) for increasing the accuracy of determining said internal temperature, wherein said step (103) comprises, prior to step (104):
- inserting a second temperature sensor for invasively measuring said internal temperature at said predetermined location in the human or animal body.

13. The method according to claim 12, wherein the method further comprises the step (106) of
- comparing the calculated internal temperature with the invasively measured internal temperature and refining said calculating of the internal temperature by correlating and/or calibrating the calculated internal temperature with the invasively measured internal temperature.

14. A computer program product for carrying out computer program product comprising code means configured to cause a processor to perform the functions of the system according to any one of claims 1-7, the thermal treatment device according to claim 8 or the method according to any one of claims 11-13.
